(19) Europäisches Patentamt
European Patent Office
Office européen des brevets

(11) Veröffentlichungsnummer: **0 170 193 B1**

# (12) EUROPÄISCHE PATENTSCHRIFT

(45) Veröffentlichungstag der Patentschrift: **27.12.91**

(51) Int. Cl.$^5$: **A23K 1/16**, A61K 9/22

(21) Anmeldenummer: **85109210.6**

(22) Anmeldetag: **23.07.85**

## (54) Futtermittelzusatz zur Verbesserung des Wachstums bei landwirtschaftlichen Nutztieren.

(30) Priorität: **01.08.84 DE 3428342**

(43) Veröffentlichungstag der Anmeldung:
**05.02.86 Patentblatt 86/06**

(45) Bekanntmachung des Hinweises auf die Patenterteilung:
**27.12.91 Patentblatt 91/52**

(84) Benannte Vertragsstaaten:
**AT BE CH DE FR GB IT LI LU NL SE**

(56) Entgegenhaltungen:
**CH-A- 630 245**
**DE-A- 2 154 049**

(73) Patentinhaber: **BOEHRINGER INGELHEIM VET-MEDICA GMBH**

**W-6507 Ingelheim/Rhein(DE)**

(72) Erfinder: **Bomann, Werner, Dr.**
**Albrecht-Dürer-Strasse 33**
**W-6507 Ingelheim am Rhein(DE)**
Erfinder: **Esser, Franz, Dr.**
**Posener Strasse 30**
**W-6507 Ingelheim am Rhein(DE)**
Erfinder: **Hamel, Ulrich, Dr.**
**Am Römergrab 9**
**W-6507 Ingelheim am Rhein(DE)**
Erfinder: **Stähle, Helmut, Dr.**
**Rotweinstrasse 23**
**W-6507 Ingelheim am Rhein(DE)**

**EP 0 170 193 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Futtermittelzusätze zur Verbesserung des Wachstums bei landwirtschaftlichen Nutztieren. Sympathomimetika vermitteln ihre Wirkungen über alpha- und beta-Rezeptoren, wobei es natürliche und synthetische Substanzen gibt, die überwiegend entweder über beta- oder alpha-Rezeptoren wirksam sind.

Die Wirkung der vorwiegend über alpha-Rezeptoren wirkenden Sympathomimetika besteht z.B. in der Kontraktion der glatten Muskulatur der Gefäße und des Uterus, der Sphinkteren im Magen-Darm-Trakt und des M. dilatator Pupillae (Mydriasis). Weiterhin wirken sie erschlaffend auf die Längsmuskulatur des Magen-Darm-Traktes und bewirken Glykogenolyse in der Leber.

Die $\alpha$-Mimetika werden systemisch zur Therapie der hypotonen Kreislaufregulation herangezogen, lokal zur Abschwellung der Nasenschleimhaut, zur Stillung von diffusen Blutungen, als Mydriaticum und als Zusatz zu Lokalanaesthetika.

Es ist bekannt, daß Clonidin bei einigen Labortierarten nach i.v.-Applikation zu einer erheblichen Steigerung der Wachstumshormon-Produktion führte und beim Affen nach i.m.-Applikation in einer Steigerung des Appetits und einer damit verbundenen Futtermehraufnahme und kurzzeitigen Gewichtszunahme resultierte. Bei der Ratte konnte ebenfalls - allerdings nach intracerebraler Gabe - die Futteraufnahme gesteigert werden. Diese Ergebnisse stammen aus pharmakologischen Versuchen, wobei erwähnt werden muß, daß insbesondere die Erhöhung der Wachstumshormon-Blutspiegel nur von relativ kurzer Dauer (einige Stunden) war.

Überraschenderweise wurde nun gefunden, daß bei Verwendung von $\alpha$-Mimetica, insbesondere von $\alpha$-Mimetica aus der Gruppe der allgemeinen Formel I - III wie auch der in Tabelle I aufgeführten Verbindungen und deren physiologisch verträgliche Säureadditionssalze, als Futterzusatz bei der Tierhaltung die tägliche Gewichtszunahme erhöht und die Futterverwertung verbessert wird.

Imidazoline der allgemeinen Formel

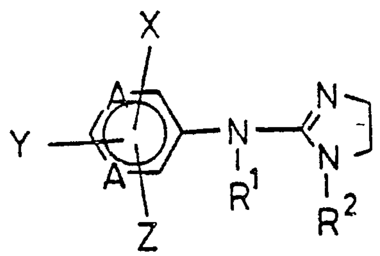

I

in der X, Y, Z gleich oder verschieden sein können, ein Wasserstoff- oder Halogenatom, wie z.B. F, Cl, Br, eine Alkyl-, Halogenalkyl-,Alkoxy-, Halogenalkoxy-, Amino-, Nitro-, Hydroxy-, Alkylthio- oder Halogenthiogruppe, oder Y und Z zusammen einen Alkylendioxyrest, einen $-N=(CH)_2=N-$-Rest, einen $-(CH_2)_n-$-Rest mit n = 4, Y einen Cyclopropylrest, A ein Kohlenstoff- oder Stickstoffatom, $R^1$ ein Wasserstoffatom, einen Alkyl- oder Tetrahydropyranrest, $R^2$ Wasserstoff,einen Benzoylrest oder einen Acetonylrest bedeuten können; oder Imidazo [1,2-a] s-triazine der allgemeinen Formel

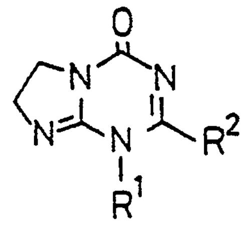

II

in der $R^1$ einen unsubstituierten oder durch Halogenatome, vorzugsweise Fluor-, Chlor-, oder Bromatome, Methyl-, Methoxy-, Trifluormethylgruppen gleich oder verschieden ein- bis dreifach substituierten Phenylrest und $R^2$ ein Wasserstoffatom oder einen unsubstituierten oder ein bis mehrfach durch Halogenatome, vorzugsweise Chloratome, substituierten Phenylrest bedeuten;

oder ein Azepinderivat der allgemeinen Formel

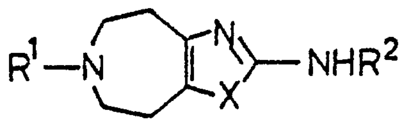 III

in der

$R^1$ ein Wasserstoffatom, einen gegebenenfalls durch eine Hydroxylgruppe substituierten geradkettigen oder verzweigten Alkylrest mit 1 - 4 Kohlenstoffatomen, einen Allyl-, Cycloalkyl-, Hexahydrobenzyl-, Phenyl-, Phenyläthyl oder Benzylrest, wobei der Benzylrest im Kern durch ein oder zwei Halogenatome, durch ein bis drei Methoxygruppen, durch eine Trifluormethyl- oder Alkylgruppe mit 1 - 3 Kohlenstoffatomen substituiert sein kann und, falls

X ein Schwefelatom darstellt,

$R_2$ ein Wasserstoffatom, einen geradkettigen oder verzweigten Alkylrest mit 1 - 5 Kohlenstoffatomen, einen Allyl-, Cycloalkyl-, Phenyl-, Benzyl- oder Phenyläthylrest oder, falls

X ein Sauerstoffatom darstellt, ein Wasserstoffatom, bedeuten,

sowie die physiologisch verträglichen Säureadditionssalze der allgemeinen Formeln I - III können erfindungsgemäß als Futterzusatz verwandt werden.

Tabelle 1:

1) 2-[1-(2,6-Dichlorphenoxy)ethyl]-2-imidazolin.

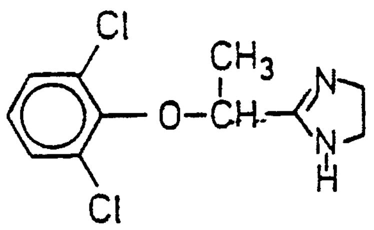

2) 2-[(2-Chlor-4-methyl-3-thienyl)amino]-2-imidazolin.

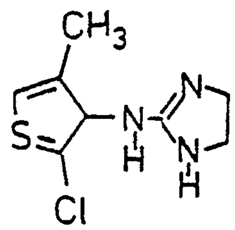

3) 2,6-Dichlorphenylacetylguanidin.

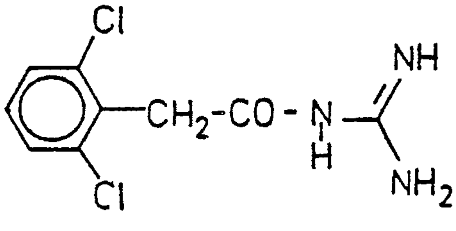

4) 1-(2,6-Dichlorbenzylidenamino)guanidin.

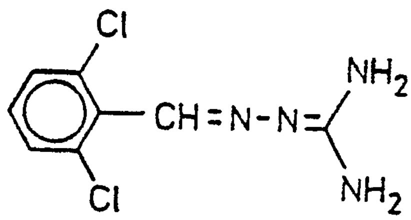

5) 2-(2,6-Dimethylbenzylamino)-4,5-dihydro-6H-1,3-thiazin.

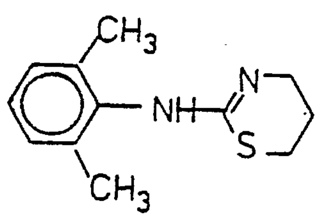

6) 2,6-Dichlorbenzaldehyd-(4-amino-4H-1,2,4-triazol-3-yl)-hydrazon.

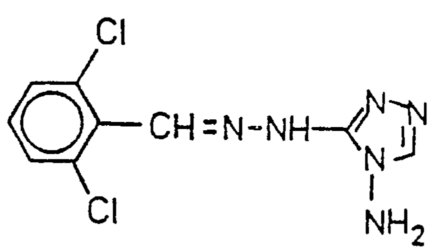

7) 2-(2,6-Dichlorphenyl)-5,6-dihydroimidazo-[2,1-b]thiazol.

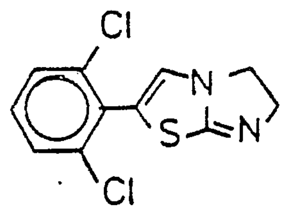

8) 4-(2,6-Dimethylbenzyl)imidazol.
1-(Imidazol-4-yl)-2-(2,6-dimethylphenyl)-ethan.
1-(Imidazol-4-yl)-3-(2,6-dimethylphenyl)-propan.
1-(Imidazol-4-yl)-4-(2,6-dimethylphenyl)-butan.

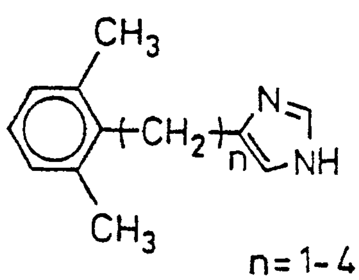

n=1-4

9) 6-(2,6-Dichlorphenyl)-2,3,6,7-tetrahydro-5H-pyrrolo-[2,1-b]imidazol.
6-(2-Chlor-6-fluorphenyl)-2,3,6,7-tetrahydro-5H-pyrrolo-[2,1-b]imidazol.
6-(2,6-Dichlor-3-methylphenyl)-2,3,6,7-tetrahydro-5H-pyrrolo[2,1-b]imidazol.

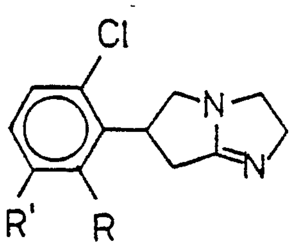

R = Cl  R' = H
R = F  R' = H
R = Cl  R' = $CH_3$

10) 2-(4-t-Butyl-2,6-dimethyl-3-hydroxy-benzyl)-2-imidazolin
2-(4-t-Butyl-2,6-dimethyl-benzyl)-2-imidazolin.

R = OH
R = H

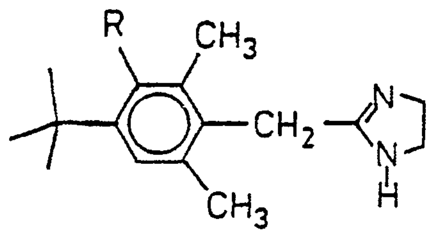

11) 2-(1,2,3,4-Tetrahydro-1-naphthyl)-2-imidazolin.

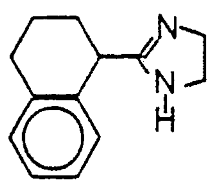

12) 2-(1'-Naphthylmethyl)-2-imidazolin.

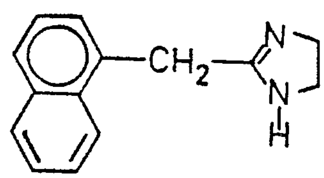

13) 2-(2-Methoxy-5-chlorphenyl)azoimidazol.

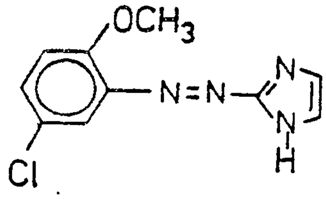

14) 2-[N-(4-Hydroxy-2-methylbenzyliden)hydrazino]-2-imidazolin.

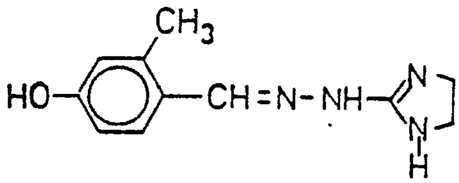

15) 2-Dimethylamino-5,6-dihydroxy-1,2,3,4-tetrahydronaphthalin.

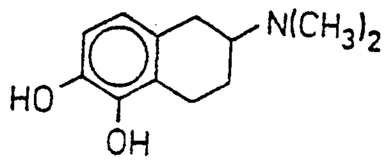

15a) 5- Chlor-4-(2-imidazolin-2-ylamino)-2,1,3-benzothiadiazol.

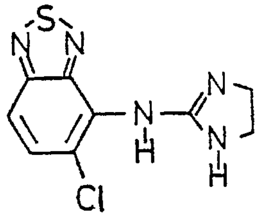

15b) 4-(2-Imidazolin-2-ylamino)-2-methyl-benzopyrazol.

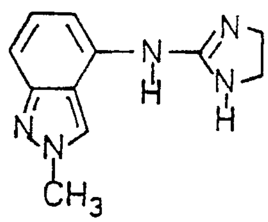

16) 2-Amino-1-(2,5-dimethoxyphenyl)-propanol.

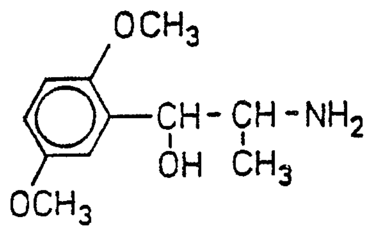

17) 1-(3-Hydroxyphenyl)-2-methylamino-ethanol.

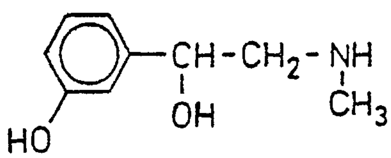

18) 2-[(o-Cyclopropylphenoxy)methyl]-2-imidazolin.

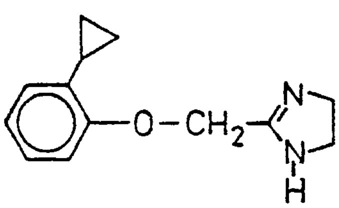

19) 3'-(1-Hydroxy-2-methylaminoethyl)methansulfonanilid.

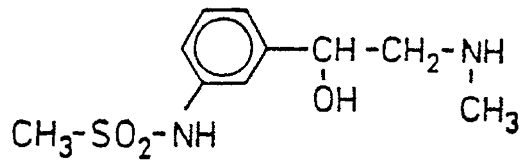

20) 2-Amino-5-methylthio-8-methoxy-1,2,3,4-tetrahydronaphthalin.

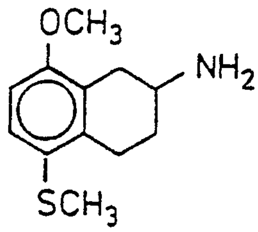

Aus der Gruppe der allgemeinen Formeln I - III sind die folgenden Verbindungen bevorzugt.

1) 2-(2,6-Dichloranilino)-2-imidazolin.
2) 8-(2,6-Dichlorophenyl)-7-(4-chlorphenyl)-5-oxo-2,3-dihydroimidazo[1,2-a]s-triazin.
3) 1-Acetonyl-2-(2,6-dichlorphenylamino)-2-imidazolin.
4) 2-(2-Brom-6-fluoranilino)-2-imidazolin.
5) 2-(2-Fluor-6-trifluormethylphenylamino)-2-imidazolin.
6) 2-(2-Chlor-5-trifluormethylphenylamino)-2-imidazolin.
7) 2-(2-Chlor-4-cyclopropylphenylamino)-2-imidazolin.
8) 2-(3-Fluor-4-methylphenylamino)-2-imidazolin.
9) 2-(6-Chlor-4-methoxy-2-methyl-pyrimidin-5-ylamino)-2 imidazolin.
10) 1-Benzoyl-2-(2,6-dichloranilino)-2-imidazolin.
11) 2-[N-(2,6-Dichlorphenyl)-N-tetrahydropyran-2-yl)-amino]-2-imidazolin.
12) 5-Chlor-4-(2-imidazolin-2-ylamino)-2,1,3-benzothiadiazol.
13) 2-(1,2,3,4-Tetrahydro-5-naphthylamino)-2-imidazolin.
14) 2-(4-Amino-2,6-dichlorphenylamino)-2-imidazolin.
15) 4-(2-Imidazolin-2-ylamino)-2-methyl-1,2-benzopyrazol
16) 2-(3,4-Dihydroxy-phenylamino)-2-imidazolin.
17) 2-Amino-6-ethyl-4,5,7,8-tetrahydro-6H-oxazolo[5,4-d]-azepin.
18) 2-Amino-6-(p-chlorbenzyl)-4,5,7,8-tetrahydro-6H-thiazolo-[5,4-d]azepin.
19) 2-(4-Brom-chinoxalin-5-ylamino)-2-imidazolin.

Unerwartet war, daß bei dem erfindungsgemäßen Zusatz von α-Mimetica, insbesondere von α-Mimetica der allgemeinen Formel I - III sowie der in der Tabelle I aufgeführten Verbindungen, die Verbesserung der Gewichtszunahme auch bei reduzierter Futteraufnahme eintrat. Die Schlachtausbeuten wurden erhöht, und der Schlachtkörper in dem Sinne verbessert, daß das Verhältnis von Muskulatur zu Fett vergrößert wurde. Die Fleischqualität wurde nicht negativ beeinflußt.

Diese Effekte bei den Nutztieren wurden durch tägliche Applikationen über das Futter oder durch parenterale Systeme mit verzögerter Wirksubstanzfreigabe erreicht.

Insbesondere überraschte, daß die beobachteten leistungsverbesserten Effekte über längere Zeiträume aufrechterhalten werden konnten, was aufgrund der zu erwartenden endokrinen gegenregulatorischen Maßnahmen nicht vorherzusehen war.

Somit konnten die o.a. Wirkungen über die für jede Tierspezies typischen Mast- bzw. Leistungsperioden voll wirksam werden.

α-Mimetica stellen also ein für die Tierproduktion wertvolles Hilfsmittel dar.

Hierzu wird erfindungsgemäß eine obige Verbindung in einer Dosierung von 0,02 - 20 ppm (Schwein), 0,08 - 80 ppm (Geflügel) und 0,02 - 200 ppm (Rind) den Alleinfuttermitteln zugefügt, so daß eine Tagesdosis von 1 - 1000 mcg/kg Körpergewicht erreicht wird.

Als Nutztiere kommen beispielsweise Schweine, Rinder, Mastgeflügel, anderes Geflügel wie Hühner, Enten, Gänse oder Truthühner, Schafe, Kaninchen und Fische in Betracht.

Als besonders geeignetes Futter, dem jeweils eine obige Verbindung oder dessen physiologisch verträgliches Säureadditionssalz in der vorstehend genannten Konzentration beigemischt war, kommt beispielsweise

für Schweine Milchaustauschfuttermittel für Ferkel, Alleinfuttermittel für Ferkel (Ferkelaufzuchtfuttermittel), Alleinfuttermittel I für Mastschweine bis etwa 50 kg, Alleinfuttermittel II für Mastschweine von etwa 50 kg an, Alleinfuttermittel für Mastschweine von etwa 35 kg an,
Ergänzungsfuttermittel für Ferkel, Ergänzungsfuttermittel I für Mastschweine, Ergänzungsfuttermittel II für Mastschweine, eiweißreiches Ergänzungsfuttermittel für Schweine oder Eiweißkonzentrat für Schweine (Ergänzungsfuttermittel), für Rinder Milchaustauschfuttermittel für Aufzuchtkälber,
Ergänzungsfuttermittel zu Magermilch für Aufzuchtkälber, Ergänzungsfuttermittel für Aufzuchtkälber, Magermilchaustauschfuttermittel I für Mastkälber, Milchaustauschfuttermittel II für Mastkälber von etwa 80 kg an, energiereiches Ergänzungsfuttermittel zu Magermilch für Mastkälber, Ergänzungsfuttermittel für Mastrinder oder eiweißreiches Ergänzungsfuttermittel für Mastrinder,
für Schafe Milchaustauschfuttermittel für Lämmer oder Alleinfuttermittel für Mastlämmer,
für Geflügel Alleinfuttermittel für Mastgänse, Ergänzungsfuttermittel für Mastgänse, Alleinfuttermittel für Entenküken, Alleinfuttermittel für Jungenten, Alleinfuttermittel für Hühnerküken in den ersten Lebenswochen, Alleinfuttermittel für Hühnerküken, Alleinfuttermittel für Junghennen, Alleinfuttermittel I für Masthühnerküken (Broiler), Alleinfuttermittel II für Masthühnerküken (Broiler) von etwa ab der 5. Lebenswoche an, Ergänzungsfuttermittel für Hühnerküken, Alleinfuttermittel für Truthühnerküken, Alleinfuttermittel für Jungtruthühner oder Alleinfuttermittel für Masthühner,
für Kaninchen Alleinfuttermittel für Mastkaninchen oder Ergänzungsfuttermittel für Mastkaninchen und für Fische Alleinfuttermittel für Karpfen oder Alleinfuttermittel für Forellen bzw. entsprechende Beifutter oder Ergänzungsfutter in Betracht.

So trägt beispielsweise die Konzentration an einer obigen Verbindung oder ihrem physiologisch verträglichen Salz bei Alleinfutter 0,02 bis 200 ppm und bei Ergänzungsfutter 0,2 bis 1000 ppm.

Der erfindungsgemäße Futterzusatz unterscheidet sich von den bisher eingesetzten antibakteriellen Substanzen, die durch Beeinflussung der Darmflora leistungsfördernd wirken, dadurch, daß er direkt die am Wachstumsgeschehen beteiligten endokrinen Regelkreise zugunsten einer anabolen Stoffwechselsituation beeinflußt.

Außerdem weist der erfindungsgemäße Futtermittelzusatz eine geringe Rückstandsproblematik auf, das heißt eine geringe Toxizität, keine hemmende Wirkung auf das Bakterienwachstum, somit keine Dysbiose und keine Resistenzbildung bzw. -übertragung.

Der erfindungsgemäße Futterzusatz unterscheidet sich von den bisher bekannten Futterzusätzen insbesondere durch die bevorzugte Wirkung auf die Verbesserung der Qualität des Schlachtkörpers, d.h. durch eine selektive Verbesserung des Muskel:Fett-Verhältnisses zugunsten des Muskel- und Proteinanteils.

Die nachfolgenden Beispiele für Futterzusammensetzungen sollen die Erfindung näher erläutern:
(Alle Mengenangaben beziehen sich auf Gewichtsprozent, sofern nichts anderes angegeben ist.)

Beispiel 1

Alleinfuttermittel I für Mastschweine bis etwa 50 kg

| | | | |
|---|---|---|---|
| a) | Rohprotein | min. | 16 |
| | Lysin | min. | 0,8 |
| | Rohfett | max. | 8 |
| | Rohfaser | max. | 6 |
| | Stärke | min. | 33 |
| | Calcium | min. | 0,7 |
| | Phosphor | min. | 0,5 |
| | Natrium | min. | 0,15 |
| b) | Kupfer | min. | 20 mg |
| | Zink | min. | 50 mg |
| | Vitamin A | min. | 4 000 IE |
| | Vitamin D | min. | 500 IE |

c) 2-(2,6-Dichloranilino)-2-imidazolin 0,02 - 20 ppm

Beispiel 2

Milchaustauschfuttermittel II für Mastkälber von etwa 80 kg an (Alleinfuttermittel)

| | | | |
|---|---|---|---|
| a) | Rohprotein | min | 17 |
| | Lysin | min. | 1,25 |
| | Rohfett | | 15 bis 30 |
| | Rohfaser | max. | 2 |
| | Rohasche | max. | 10 |
| | Calcium | min. | 0,9 |
| | Phosphor | min. | 0,7 |
| | Natrium | min. | 0,2 |
| | Magnesium | min. | 0,13 |
| | Milchpulver | min. | 25 |

| | | | |
|---|---|---|---|
| b) Kupfer | max. | 15 mg | |
| Vitamin A | min. | 8 000 IE | |
| Vitamin D | min. | 1 000 IE | |
| Vitamin E | min. | 20 mg | |
| c) 2-(2,6-Dichloranilino)-2-imidazolin | | | 0,02 - 200 ppm |

Beispiel 3

Rindermastfutter II (Eiweißreiches Ergänzungsfuttermittel für Mastrinder

| | | |
|---|---|---|
| a) Rohprotein | | 30 bis 40 |
| darunter: Rohprotein aus NPN-Verbindungen | max. | 10 |
| Rohfett | max. | 10 |
| Rohfaser | max. | 12 |
| Rohasche | max. | 12 |
| Calcium | | 1,6 bis 2,4 |
| b) 2-(2,6-Dichloranilino)-2-imidazolin | | 0,02 - 200 ppm |

Beispiel 4

Alleinfuttermittel I für Masthühnerküken (Broiler)

| | | |
|---|---|---|
| a) Rohprotein | min. | 22 |
| Methionin | min. | 0,45 |
| Gesamtzucker | max. | 12 |
| Calcium | | 0,7 bis 1,2 |
| Phosphor | min. | 0,6 |
| Natrium | | 0,12 bis 0,3 |

| | | | |
|---|---|---|---|
| b) Mangan | min. | 50 mg | |
| Zink | min. | 50 mg | |
| Vitamin A | min. | 6 000 IE | |
| Vitamin $D_3$ | min. | 750 IE | |
| Riboflavin (Vitamin $B_2$) | min. | 4 mg | |
| Vitamin $B_{12}$ | min. | 10 ug | |
| c) 2-(2,6-Dichloranilino)-2- imidazolin | | | 0,08 - 80 ppm |

Beispiel 5

Alleinfuttermittel für Mastschaflämmer

| | | | |
|---|---|---|---|
| a) Rohprotein | min. | 16 | |
| Rohfaser | max. | 8 | |
| Rohasche | max. | 9 | |
| Calcium | min. | 1 | |
| Phosphor | min. | 0,5 | |
| (Ca:P Verhältnis | nicht | unter 2:1) | |
| b) Vitamin A | min. | 10 000 IE | |
| Vitamin D | min. | 1 250 IE | |
| Vitamin E | min. | 12 mg | |
| c) 2-(2,6-Dichloranilino)-2-imidazolin | | | 0,02 - 200 ppm |

Beispiel 6

Alleinfuttermittel für Masttruthühner

| | | | |
|---|---|---|---|
| a) | Rohprotein | min. | 14 |
| | Methionin, bezogen auf Rohprotein | min. | 2 |
| | Gesamtzucker | max. | 12 |
| | Calcium | | 0,7 bis 1,7 |
| | Phosphor | min. | 0,7 |
| | Natrium | | 0,12 bis 0,3 |
| b) | Mangan | min. | 50 mg |
| | Zink | min. | 50 mg |
| | Vitamin A | min. | 8 000 IE |
| | Vitamin $D_3$ | min. | 1 000 IE |
| | Riboflavin (Vitamin $B_2$) | min. | 4 mg |
| | Biotin | min. | 0,15 mg |
| c) | 2-(2,6-Dichloranilino)-2-imidazolin | | 0,08 - 80 ppm |

## Patentansprüche

1. Verwendung von α-Mimetica und deren physiologisch verträgliche Säureadditionssalze als Futtermittelzusatz in der Tiermast zur Verbesserung des Wachstums und der Futterverwertung.

2. Verwendung von Verbindungen der allgemeinen Formel

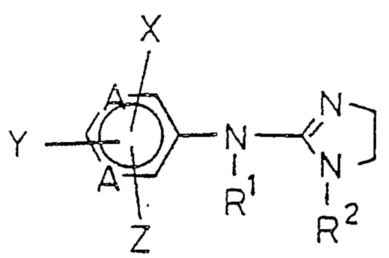

I

in der X, Y, Z gleich oder verschieden sein können, ein Wasserstoff- oder Halogenatom, wie z.B. F, Cl, Br, eine Alkyl-, Halogenalkyl-,Alkoxy-, Halogenalkoxy-, Amino-, Nitro-, Hydroxy-, Alkylthio- oder Halogenthiogruppe, oder Y und Z zusammen einen Alkylendioxyrest, einen $-N=(CH)_2=N-$-Rest, einen $-(CH_2)_n-$-Rest mit n=4, Y einen Cyclopropylrest, A ein Kohlenstoff- oder Stickstoffatom, $R^1$ ein Wasserstoffatom, einen Alkyl- oder Tetrahydropyranrest, $R^2$ Wasserstoff einen Benzoylrest oder einen Acetonylrest bedeuten können; oder Imidazo [1,2-a] s-triazine der allgemeinen Formel

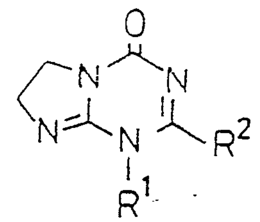

II

in der $R^1$ einen unsubstituierten oder durch Halogenatome, vorzugsweise Fluor-, Chlor-, oder Bromatome, Methyl-, Methoxy-, Trifluormethylgruppen gleich oder verschieden ein bis dreifach substituierten Phenylrest und $R^2$ ein Wasserstoffatom oder einen unsubstituierten oder ein bis mehrfach durch Halogenatome, vorzugsweise Chloratome, substituierten Phenylrest bedeuten;
oder ein Azepinderivat der allgemeinen Formel

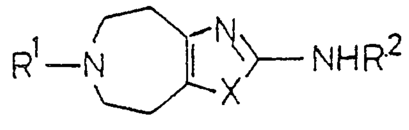

III

in der
$R^1$ ein Wasserstoffatom, einen gegebenenfalls durch eine Hydroxylgruppe substituierten geradkettigen oder verzweigten Alkylrest mit 1 - 4 Kohlenstoffatomen, einen Allyl-, Cycloalkyl-, Hexahydrobenzyl-, Phenyl-, Phenyläthyl- oder Benzylrest, wobei der Benzylrest im Kern durch ein oder zwei Halogenatome, durch ein bis drei Methoxygruppen, durch eine Trifluormethyl- oder Alkylgruppe mit 1 - 3 Kohlenstoffatomen substituiert sein kann und, falls
X ein Schwefelatom darstellt,
$R_2$ ein Wasserstoffatom, einen geradkettigen oder verzweigten Alkylrest mit 1 - 5 Kohlenstoffatomen, einen Allyl-, Cycloalkyl-, Phenyl-, Benzyl- oder Phenyläthylrest oder, falls
X ein Sauerstoffatom darstellt, ein Wasserstoffatom, bedeuten,
sowie die physiologisch verträglichen Säureadditionssalze der allgemeinen Formeln I - III nach Anspruch 1 als Futtermittelzusatz in der Tiermast zur Verbesserung des Wachstums und der Futterverwertung.

3. Verwendung der folgenden Verbindungen:
1) 2-[1-(2,6-Dichlorphenoxy)ethyl]-2-imidazolin.

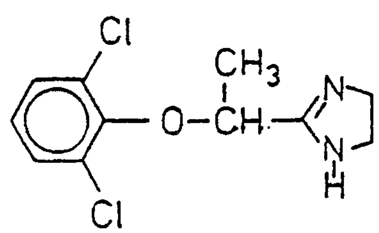

2) 2-[(2-Chlor-4-methyl-3-thienyl)amino]-2-imidazolin.

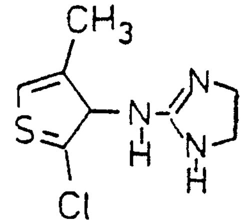

3) 2,6-Dichlorphenylacetylguanidin.

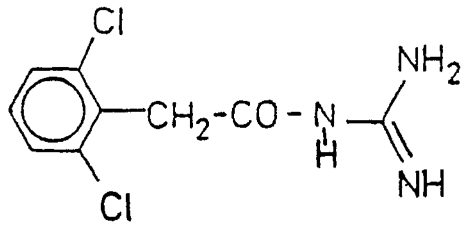

4) 1-(2,6-Dichlorbenzylidenamino)guanidin.

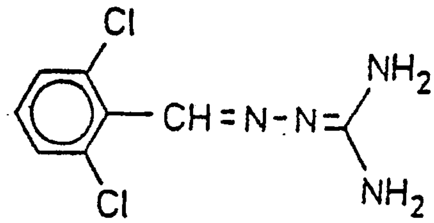

5) 2-(2,6-Dimethylbenzylamino)-4,5-dihydro-6H-1,3-thiazin.

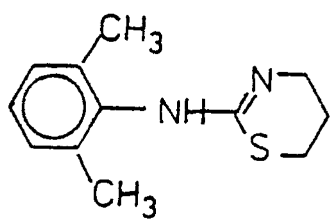

6) 2,6-Dichlorbenzaldehyd-(4-amino-4H-1,2,4-triazol-3-yl)-hydrazon.

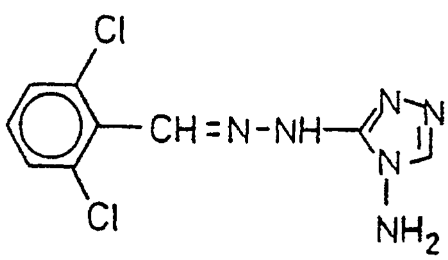

7) 2-(2,6-Dichlorphenyl)-5,6-dihydroimidazo-[2,1-b]thiazol.

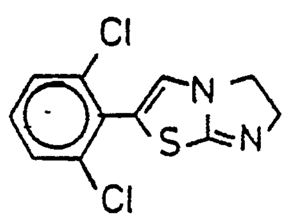

8) 4-(2,6-Dimethylbenzyl)imidazol.
1-(Imidazol-4-yl)-2-(2,6-dimethylphenyl)-ethan.
1-(Imidazol-4-yl)-3-(2,6-dimethylphenyl)-propan.

1-(Imidazol-4-yl)-4-(2,6-dimethylphenyl)-butan.

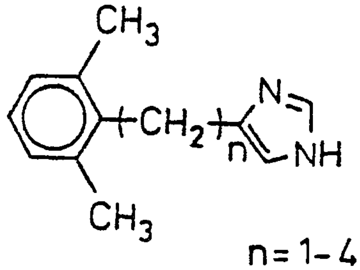

n = 1-4

9) 6-(2,6-Dichlorphenyl)-2,3,6,7-tetrahydro-5H-pyrrolo-[2,1-b]imidazol.
6-(2-Chlor-6-fluorphenyl)-2,3,6,7-tetrahydro-5H-pyrrolo-[2,1-b]imidazol.
6-(2,6-Dichlor-3-methylphenyl)-2,3,6,7-tetrahydro-5H-pyrrolo[2,1-b]imidazol.

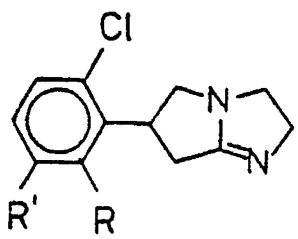

R = Cl  R' = H
R = F  R' = H
R = Cl  R' = $CH_3$

10) 2-(4-t-Butyl-2,6-dimethyl-3-hydroxy-benzyl)-2-imidazolin
2-(4-t-Butyl-2,6-dimethyl-benzyl)-2-imidazolin.

R = OH
R = H

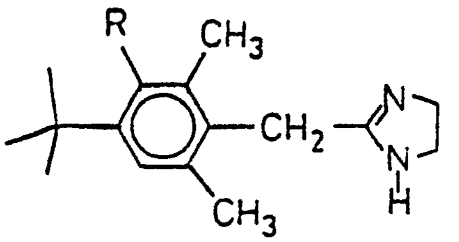

11) 2-(1,2,3,4-Tetrahydro-1-naphthyl)-2-imidazolin.

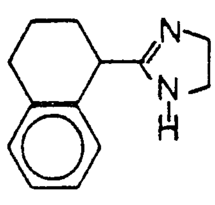

12) 2-(1'-Naphthylmethyl)-2-imidazolin.

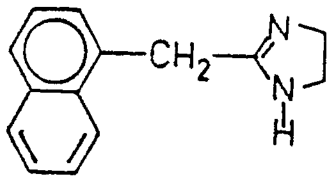

13) 2-(2-Methoxy-5-chlorphenyl)azoimidazol.

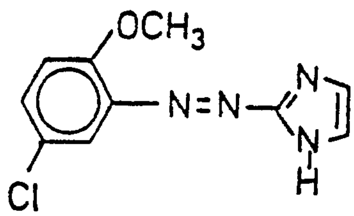

14) 2-[N-(4-Hydroxy-2-methylbenzyliden)hydrazino]-2-imidazolin.

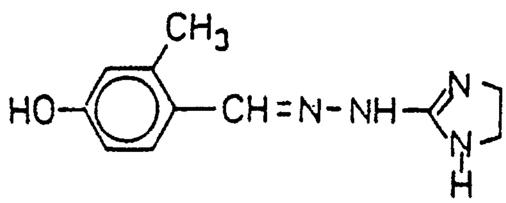

15) 2-Dimethylamino-5,6-dihydroxy-1,2,3,4-tetrahydronaphthalin.

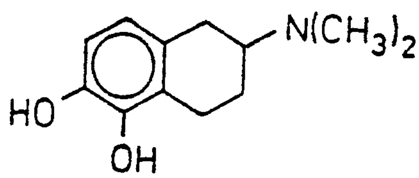

15a) 5- Chlor-4-(2-imidazolin-2-ylamino)-2,1,3-benzothiadiazol.

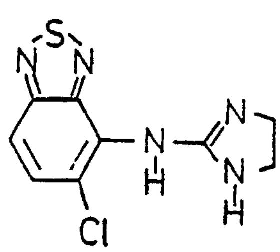

15b) 4-(2-Imidazolin-2-ylamino)-2-methyl-benzopyrazol.

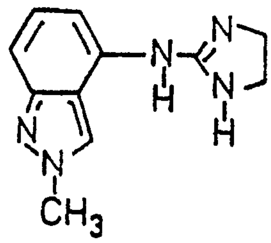

16) 2-Amino-1-(2,5-dimethoxyphenyl)-propanol.

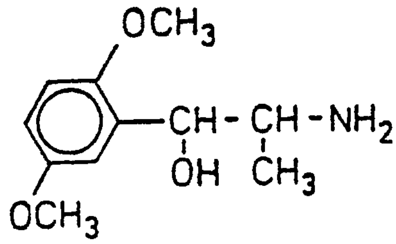

17) 1-(3-Hydroxyphenyl)-2-methylamino-ethanol.

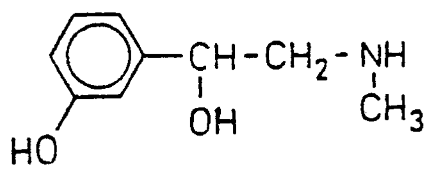

18) 2-[(O-Cyclopropylphenoxy)methyl]-2-imidazolin.

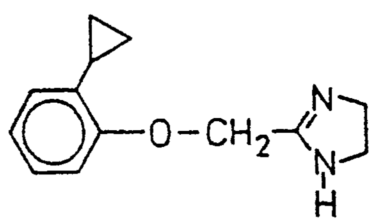

19) 3'-(1-Hydroxy-2-methylaminoethyl)methansulfonanilid.

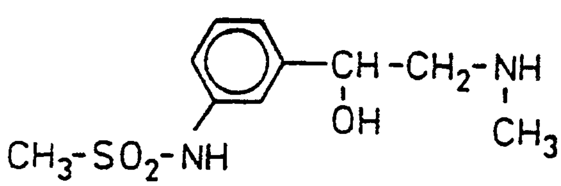

20) 2-Amino-5-methylthio-8-methoxy-1,2,3,4-tetrahydronaphthalin.

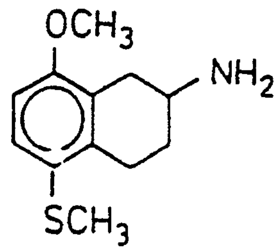

sowie deren physiologisch verträgliche Säureadditionssalze nach Anspruch 1 in der Tiermast zur Verbesserung des Wachstums und der Futterverwertung.

4. Verwendung einer oder mehrere der folgenden Verbindungen nach Anspruch 2.
1) 2-(2,6-Dichloranilino)-2-imidazolin,
2) 8-(2,6-Dichlorphenyl)-7-(4-chlorphenyl)-5-oxo-2,3-dihydroimidazo[1,2-a]s-triazin,
3) 1-Acetonyl-2-(2,6-dichlorphenylamino)-2-imidazolin,
4) 2-(2-Brom-6-fluoranilino)-2-imidazolin,
5) 2-(2-Fluor-6-trifluormethylphenylamino)-2-imidazolin,
6) 2-(2-Chlor-5-trifluormethylphenylamino)-2-imidazolin,
7) 2-(2-Chlor-4-cyclopropylphenylamino)-2-imidazolin,
8) 2-(3-Fluor-4-methylphenylamino)-2-imidazolin,
9) 2-(6-Chlor-4-methoxy-2-methyl-pyrimidin-5-ylamino)-2-imidazolin,
10) 1-Benzoyl-2-(2,6-dichloranilino)-2-imidazolin,
11) 2-[N-(2,6-Dichlorphenyl)-N-tetrahydropyran-2-yl)-amino]-2-imidazolin,
12) 5-Chlor-4-(2-imidazolin-2-ylamino)-2,1,3-benzothiadiazol,
13) 2-(1,2,3,4-Tetrahydro-5-naphthylamino)-2-imidazolin,
14) 2-(4-Amino-2,6-dichlorphenylamino)-2-imidazolin,
15) 4-(2-Imidazolin-2-ylamino)-2-methyl-1,2-benzopyrazol,
16) 2-(3,4-Dihydroxy-phenylamino)-2-imidazolin,
17) 2-Amino-6-ethyl-4,5,7,8-tetrahydro-6H-oxazolo[5,4-d]-azepin,
18) 2-Amino-6-(p-chlorbenzyl)-4,5,7,8-tetrahydro-6H-thiazolo-[5,4-d]azepin,
19) 2-(4-Brom-chinoxalin-5-ylamino)-2-imidazolin.

sowie deren physiologisch verträgliche Säureadditionssalze als Futtermittelzusatz in der Tiermast zur Verbesserung des Wachstums und der Futterverwertung.

5. Verfahren zur Herstellung eines neuen Futtermittels, dadurch gekennzeichnet, daß ein übliches Futtermittel mit einer Verbindung gemäß den Ansprüchen 1 bis 4 zu einer Konzentration von 0,02 bis 1000 ppm vermischt wird.

6. Verwendung einer Verbindung gemäß den Ansprüchen 1 - 4 über parenterale Systeme mit verzögerter Wirksubstanzfreigabe zur Erhöhung der täglichen Gewichtszunahme und zur Verbesserung der Futterverwertung in der Tiermast, wobei Dosierungen von 1 bis 1000 µg/kg Körpergewicht pro Tag erreicht werden.

7. Futtermittel, enthaltend eine Verbindung gemäß den Ansprüchen 1 bis 4 in einer Konzentration von 0,02 bis 1000 ppm.

## Claims

1. Use of α-mimetics and the physiologically acceptable acid addition salts thereof as a feed additive in fattening animals for improving growth and the utilisation of fodder.

2. Use of compounds of general formula

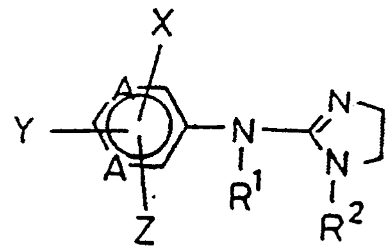

I

wherein X, Y and Z may be identical or different and may represent a hydrogen or halogen atom, such as F, Cl, Br, an alkyl, haloalkyl, alkoxy, haloalkoxy, amino, nitro, hydroxy, alkylthio or halothio group, or Y and Z together represent an alkylenedioxy group, an $-N=(CH)_2=N-$ group, a $-(CH_2)_n-$ group wherein $n=4$, Y represents a cyclopropyl group, A represents a carbon or nitrogen atom, $R^1$ represents a hydrogen atom or an alkyl or tetrahydropyrane group, $R^2$ represents hydrogen, a benzoyl or an acetonyl group; or
imidazo[1,2-a]s-triazines of general formula

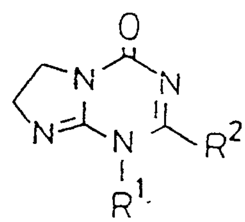

II

wherein $R^1$ represents an unsubstituted phenyl group or a phenyl group which is mono- to tri-substituted by halogen atoms, preferably fluorine, chlorine or bromine atoms or by methyl, methoxy or trifluoromethyl groups, the substituents being either identical or different, and $R^2$ represents a hydrogen atom or an unsubstituted phenyl group or a phenyl group which is mono- or poly-substituted by halogen atoms, preferably chlorine atoms;
or an azepine derivative of general formula

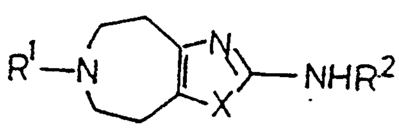

III

wherein

$R^1$ represents a hydrogen atom, a straight- chained or branched alkyl group containing 1 - 4 carbon atoms which may optionally be substituted by a hydroxyl group, an allyl, cycloalkyl, hexahydrobenzyl, phenyl, phenylethyl or benzyl group, whilst the benzyl group may be substituted in the nucleus by one or two halogen atoms, by one to three methoxy groups, by a trifluoromethyl group or an alkyl group with 1 - 3 carbon atoms and, if

X represents a sulphur atom,

$R_2$ represents a hydrogen atom, a straight-chained or branched alkyl group with 1 - 5 carbon atoms, an allyl, cycloalkyl, phenyl, benzyl or phenylethyl group or, if

X represents an oxygen atom, $R_2$ may represent a hydrogen atom,

and the physiologically acceptable acid addition salts of general formulae I - III according to claim 1 as a feed additive in fattening animals to improve growth and the utilization of fodder.

**3.** Use of the following compounds:

1) 2-[1-(2,6-Dichlorophenoxy)ethyl]-2-imidazoline.

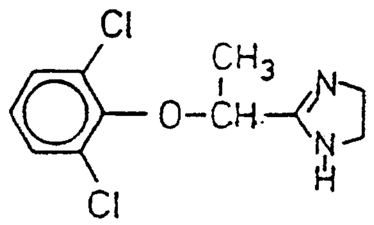

2) 2-[(2-Chloro-4-methyl-3-thienyl)amino]-2-imidazoline

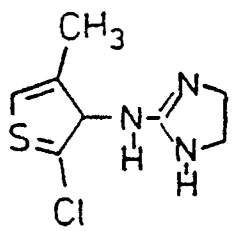

3) 2,6-Dichlorophenylacetylguanidine.

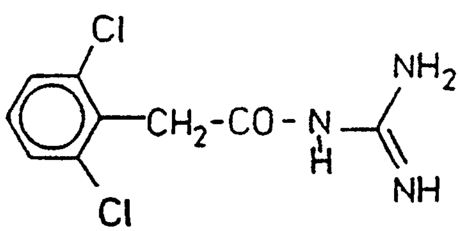

4) 1-(2,6-Dichlorobenzylideneamino)guanidine.

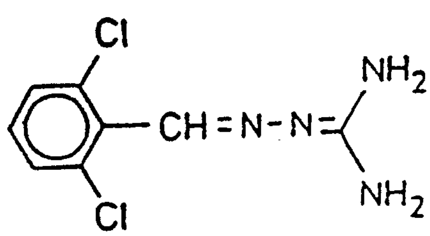

5) 2-(2,6-Dimethylbenzylamino)-4,5-dihydro-6H-1,3-thiazine.

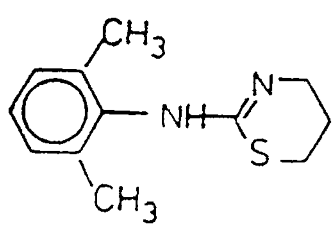

6) 2,6-Dichlorobenzaldehyde-(4-amino-4H-1,2,4-triazol-3-yl)-hydrazone.

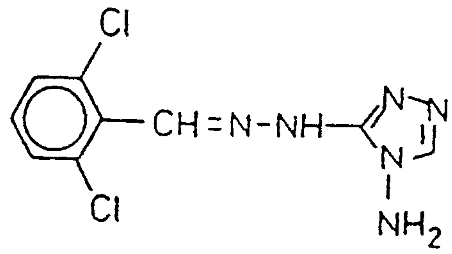

7) 2-(2,6-Dichlorophenyl)-5,6-dihydroimidazo-[2,1-b]-thiazole.

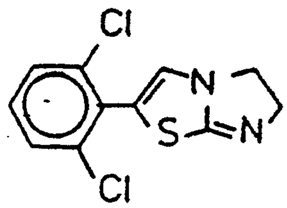

8) 4-(2,6-Dimethylbenzyl)imidazole.
1-(Imidazol-4-yl)-2-(2,6-dimethylphenyl)-ethane.
1-(Imidazol-4-yl)-3-(2,6-dimethylphenyl)-propane.
1-(Imidazol-4-yl)-4-(2,6-dimethylphenyl)-butane.

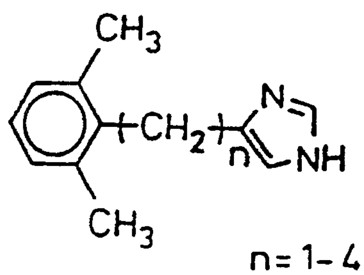

n= 1- 4

9) 6-(2,6-Dichlorophenyl)-2,3,6,7-tetrahydro-5H-pyrrolo[2,1-b]imidazole.
6-(2-Chloro-6-fluorophenyl)-2,3,6,7-tetrahydro-5H-pyrrolo(2,1-b]imidazole.
6-(2,6-Dichloro-3-methylphenyl)-2,3,6,7-tetrahydro-5H-pyrrolo[2,1-b]imidazole.

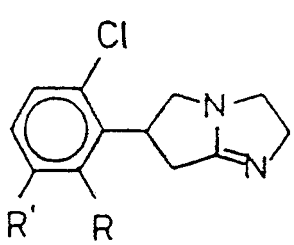

R = Cl R' = H
R = F R' = H
R = Cl R' = $CH_3$

10) 2-(4-t-Butyl-2,6-dimethyl-3-hydroxy-benzyl)-2-imidazoline
2-(4-t-Butyl-2,6-dimethyl-benzyl)-2-imidazoline.

R = OH
R = H

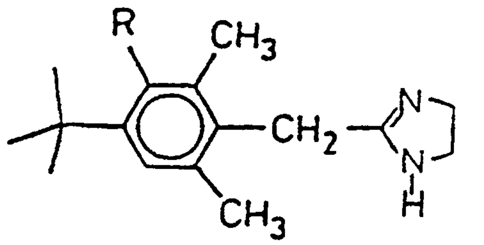

11) 2-(1,2,3,4-Tetrahydro-1-naphthyl)-2-imidazoline.

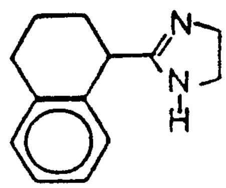

12) 2-(1'-Naphthylmethyl)-2-imidazoline.

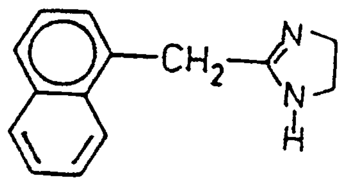

13) 2-(2-Methoxy-5-chlorophenyl)azoimidazole.

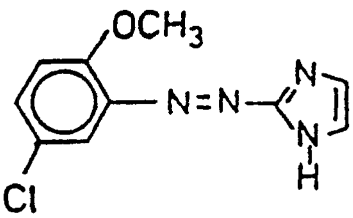

14) 2-(N-(4-Hydroxy-2-methylbenzylidene)hydrazino]-2-imidazoline.

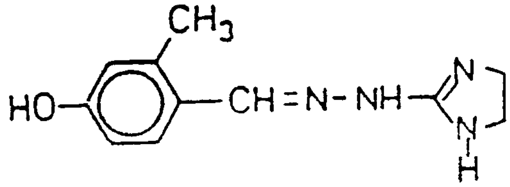

15) 2-Dimethylamino-5,6-dihydroxy-1,2,3,4-tetrahydronaphthalene.

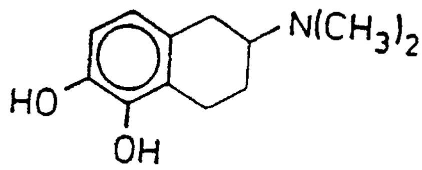

15a) 5-chloro-4-(2-imidazolin-2-ylamino)-2,1,3-benzothiadiazole.

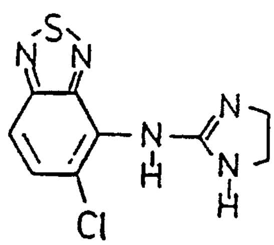

15b) 4-(2-Imidazolin-2-ylamino)-2-methyl-benzopyrazole.

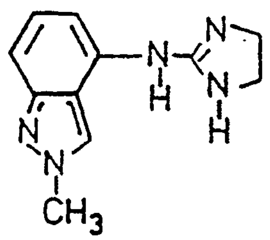

16) 2-Amino-1-(2,5-dimethoxyphenyl)-propanol.

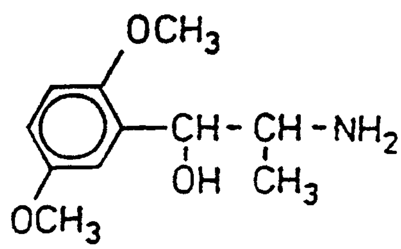

17) 1-(3-Hydroxyphenyl)-2-methylamino-ethanol.

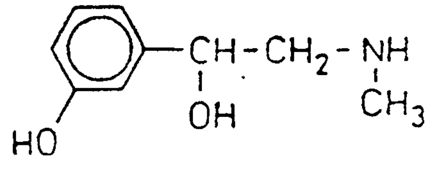

18) 2-[(O-Cyclopropylphenoxy)methyl]-2-imidazoline.

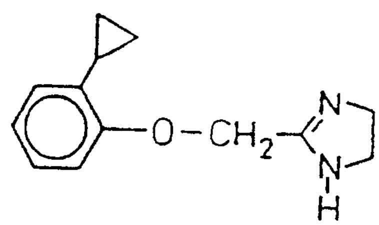

19) 3'-(1-Hydroxy-2-methylaminoethyl)methanesulphonanilide.

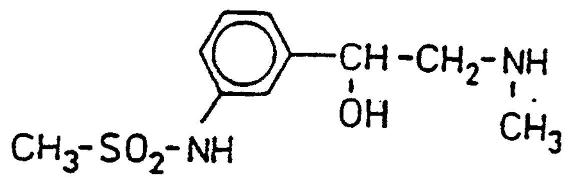

20) 2-Amino-5-methylthio-8-methoxy-1,2,3,4-tetrahydronaphthalene.

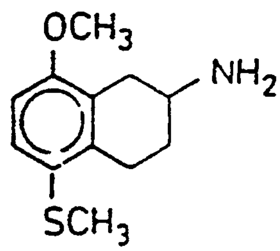

and the physiologically acceptable acid addition salts thereof according to claim 1 in fattening animals for improving growth and the utilization of fodder.

**4.** Use of one or more of the following compounds according to claim 2:

1) 2-(2,6-Dichloroanilino)-2-imidazoline,
2) 8-(2,6-Dichlorophenyl)-7-(4-chlorophenyl)-5-oxo-2,3-dihydro-imidazo[1,2-a]s-triazine,
3) 1-Acetonyl-2(2,6-dichlorophenylamino)-2-imidazoline,
4) 2-(2-Bromo-6-fluoroanilino)-2-imidazoline,
5) 2-(2-Fluoro-6-trifluoromethylphenylamino)-2-imidazoline,
6) 2-(2-Chloro-5-trifluoromethylphenylamino)-2-imidazoline,
7) 2-(2-Chloro-4-cyclopropylphenylamino)-2-imidazoline,
8) 2-(3-Fluoro-4-methylphenylamino)-2-imidazoline,
9) 2-(6-Chloro-4-methoxy-2-methyl-pyrimidin-5-yl-amino)-2-imidazoline,
10) 1-Benzoyl-2-(2,6-dichloroanilino)-2-imidazoline,
11) 2-[N-(2,6-Dichlorophenyl)-N-tetrahydropyran-2-yl)-amino]-2-imidazoline,
12) 5-Chloro-4-(2-imidazolin-2-yl-amino)-2,1,3-benzothiadiazole,
13) 2-(1,2,3,4-Tetrahydro-5-naphthylamino)-2-imidazoline,
14) 2-(4-Amino-2,6-dichlorophenylamino)-2-imidazoline,
15) 4-(2-Imidazolin-2-yl-amino)-2-methyl-1,2-benzopyrazole,
16) 2-(3,4-Dihydroxy-phenylamino)-2-imidazoline,
17) 2-Amino-6-ethyl-4,5,7,8-tetrahydro-6H-oxazolo[5,4-d]-azepine,
18) 2-Amino-6-(p-chlorobenzyl)-4,5,7,8-tetrahydro-6H-thiazolo[5,4-d]azepine,
19) 2-(4-Bromo-quinoxalin-5-yl-amino)-2-imidazoline,

and the physiologically acceptable acid addition salts thereof as feed additives in fattening animals in order to improve growth and the utilization of fodder.

**5.** Process for preparing a new feed, characterized in that a conventional feed is mixed with a compound as claimed in claims 1 to 4 to give a concentration of from 0.02 to 1000 ppm.

6. Use of a compound according to claims 1-4 by means of parenteral systems with delayed release of the active substance, for increasing daily weight gain and improving feed utilization in the fattening of animals, achieving dosages of 1 to 1000µg/kg of body weight per day.

7. Fodders, containing a compound as claimed in claims 1 to 4 in a concentration of from 0.02 to 1000 ppm.

**Revendications**

1. Utilisation de substances α-mimétiques et leurs sels d'addition d'acide compatibles physiologiquement en tant qu'additifs de matières fourragères dans l'alimentation du bétail pour l'amélioration de la croissance du bétail et de la mise en voleur du fourrage.

2. Utilisation des composés de la formule générale

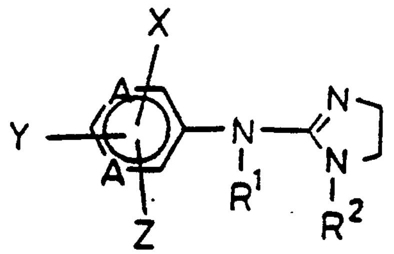

(I)

dans laquelle X, Y, Z peuvent être égaux ou différents, peuvent signifier un atome d'hydrogène ou d'halogène, comme par exemple F, Cl, Br, un groupe alkyle, alkyle halogéné, alcoxy, alcoxy halogéné, amino, nitro, hydroxy, thioalkyle ou thio halogéné, ou Y et Z conjointement peuvent signifier un radical alkylène-dioxy, un radical $-N=(CH)_2=N-$, un radical $-(CH_2)_n$-- avec n = 4, Y un radical cyclopropyle, A un atome de carbone ou d'azote, $R^1$ un atome d'hydrogène, un radical alkyle ou tétrahydropyranne, $R^2$ hydrogène, un radical benzoyle ou un radical acétonyle ; ou signifie imidazo [1,2-a] s-triazine de la formule générale

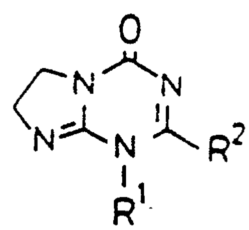

(II)

dans laquelle $R^1$ signifie un radical phényle non substitué ou substitué par des atomes d'halogène, de préférence des atomes de fluor, de chlore ou de brome, des groupes méthyle, méthoxy, trifluorométhyle identiques ou similaires substitués une à trois fois et $R^2$ signifie un atome d'hydrogène ou un radical phényle non substitué ou substitué une à plusieurs fois par des atomes d'halogène, de préférence des atomes de chlore ;

ou un dérivé d'azépine de la formule générale

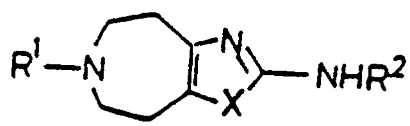

(III)

dans laquelle

$R^1$ représente un atome d'hydrogène, un radical alkyle à chaîne droite ou ramifiée avec 1-4 atomes de carbone, éventuellement substitué par un groupe hydroxyle, un radical allyle, cycloalkyle, hexahydrobenzyle, phényle, phényléthyle ou benzyle, le radical benzyle dans le noyau pouvant étre substitué par un ou deux atomes d'halogène, par un à trois groupes méthoxy, par un groupe trifluorométhyle ou alkyle avec 1-3 atomes de carbone, au cas où

X représente un atome de soufre,

$R^2$ représente un atome d'hydrogène, un radical alkyle en chaîne linéaire ou ramifiée avec 1-5 atomes de carbone, un radical allyle, cycloalkyle, phényle, benzyle ou phényléthyle,

au cas où

X représente un atome d'oxygène, ainsi que les sels d'addition d'acide physiologiquement compatibles des formules I-III selon la revendication 1 en tant qu'additifs de matières fourragères dans l'engraissement du bétail pour l'amélioration de la croissance du bétail et de la mise en valeur du fourrage.

**3.** Utilisation des composés suivants :

1) 2-[1-(2,6-dichlorophénoxy)éthyl]-2-imidazoline.

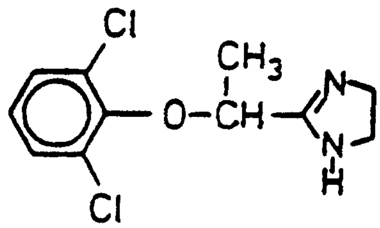

2) 2-[(2-chlor-4-méthyl-3-thiényl)amino]-2-imidazoline.

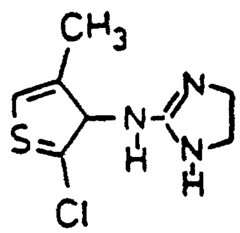

3) 2,6-dichlorophénylactylguanidine.

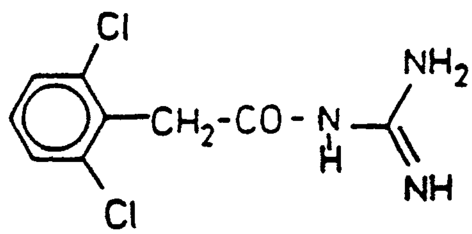

4) 1-(2,6-dichlorobenzylidène-amino)guanidine.

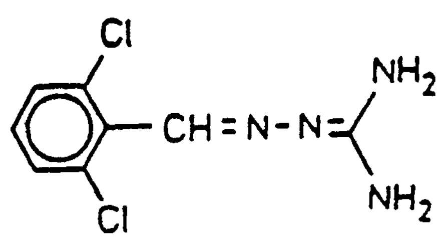

5) 2-(2,6-diméthylbenzylamino)-4,5-dihydro-6H-1,3-thiazine.

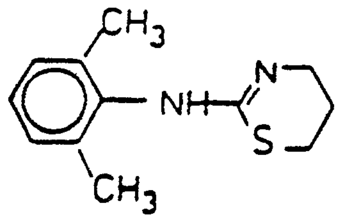

6) 2,6-dichlorobenzaldéhyd-(4-amino-4H-1,2,4-triazol-3-yl)-hydrazone.

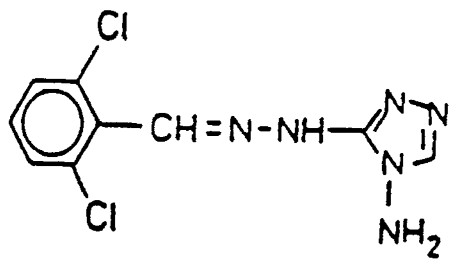

7) 2-(2,6-dichlorophényl)-5,6-dihydroimidazo-[2,1-b] thiazole.

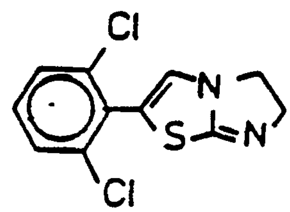

8) 4-(2,6-diméthylbenzyl)imidazole.
1-(imidazol-4-yl)-2-(2,6-diméthylphényl)-éthane.
1-(imidazol-4-yl)-3-(2,6-diméthylphényl)-propane.
1-(imidazol-4-yl)-4-(2,6-diméthylphényl)-butane.

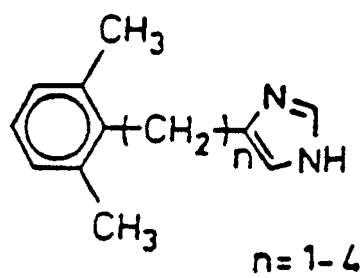

n= 1- 4

9) 6-(2,6-dichlorophényl)-2,3,6,7-tétrahydro-5H-pyrrolo-[2,1-b]imidazole.
6-(2-chlor-6-fluorophényl)-2,3,6,7-tétrahydro-5H-pyrrolo-[2,1-b]imidazole.
6-(2,6-dichlor-3-méthylphényl)-2,3,6,7-tétrahydro-5H-pyrrolo[2,1-b]imidazole.

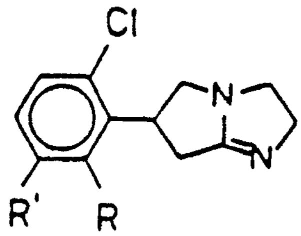

R = Cl R' = H
R = F R' = H
R = Cl R' = $CH_3$

10) 2-(4-t-butyl-2,6-diméthyl-3-hydroxy-benzyl)-2-imidazoline
2-(4-t-butyl-2,6-diméthyl-benzyl)-2-imidazoline.

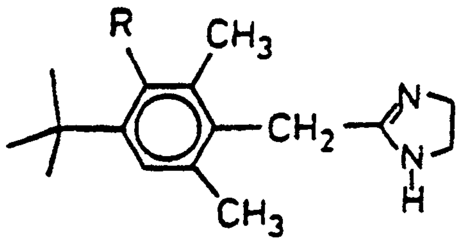

R = OH
R = H

11) 2-(1,2,3,4-tétrahydro-1-naphtyl)-2-imidazoline.

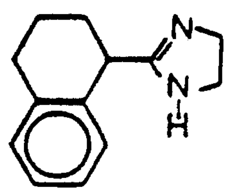

12) 2-(1'-naphtylméthyl)-2-imidazoline.

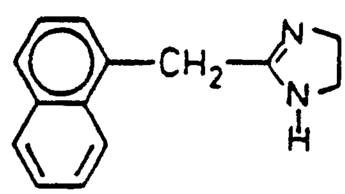

13) 2-(2-méthoxy-5-chlorophényl)azoimidazole.

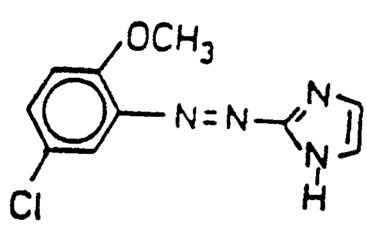

14) 2-[N-(4-hydroxy-2-méthylbenzyliden)hydrazino]-2-imidazoline.

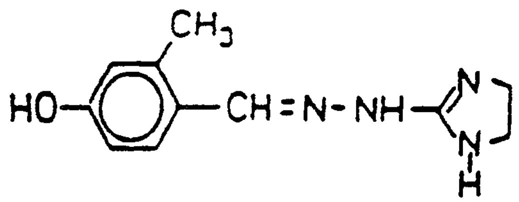

15) 2-diméthylamino-5,6-dihydroxy-1,2,3,4-tétrahydronaphtaline.

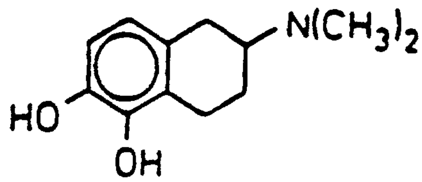

15a) 5- chlor-4-(2-imidozoline-2-ylamino)-2,1,3-benzothiadiazole.

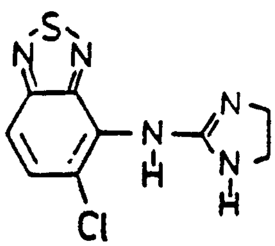

15b) 4-(2-imidazoline-2-yl-amino)-2-méthyl-benzopyrazole.

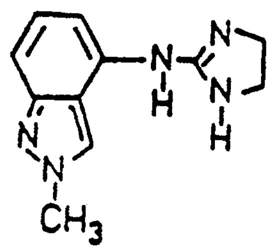

16) 2-amino-1-(2,5-diméthoxyphényl)-propanol.

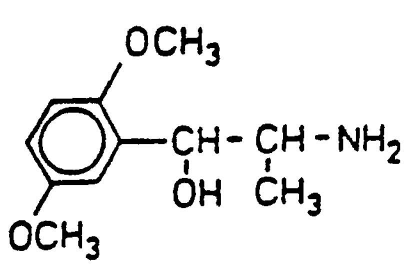

17) 1-(3-hydroxyphényl)-2-méthylamino-éthanol.

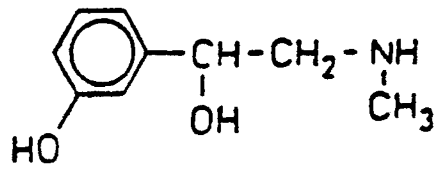

18) 2-[(O-cyclopropylphénoxy)méthyl]-2-imidazoline.

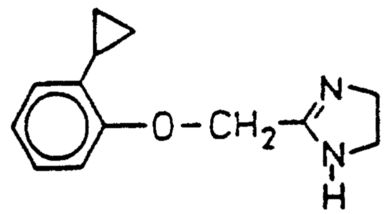

19) 3'-(1-hydroxy-2-méthylaminoéthyl)méthansulfonanilide.

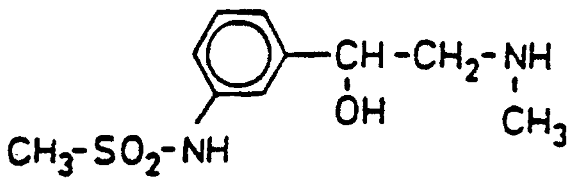

20) 2-amino-5-méthylthio-8-méthoxy-1,2,3,4-tétrahydro naphtaline.

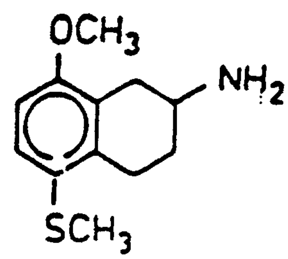

ainsi que leurs sels d'addition d'acide compatibles physiologiquement selon la revendication 1 dans l'engraissement du bétail pour l'amélioration de la croissance du bétail et de la mise en valeur du fourrage.

4. Utilisation d'un ou plusieurs des composés suivants selon la revendication 2.

1) 2-(2,6-dichloranilino)-2-imidazoline,
2) 8-(2,6-dichlorophényl)-7-(4-chlorophényl)-5-oxo-2,3-dihydro-imidazo[1,2-a]s-triazine,
3) 1-acétonyl-2-(2,6-dichlorophénylamino)-2-imidazoline,
4) 2-(2-brom-6-fluoro-anilino)-2-imidazoline,
5) 2-(2-fluor-6-trifluorométhylphénylamino)-2-imidazoline,
6) 2-(2-chlor-5-trifluorométhylphénylamino)-2-imidazoline,
7) 2-(2-chlor-4-cyclopropylphénylamino)-2-imidazoline,
8) 2-(3-fluor-4-méthylphénylamino)-2-imidazoline,
9) 2-(6-chlor-4-méthoxy-2-méthyl-pyrimidin-5-ylamino)-2-imidazoline,
10) 1-benzoyl-2-(2,6-dichloranilino)-2-imidazoline,
11) 2-[N-(2,6-dichlorophényl)-N-tétrahydropyran-2-yl)-amino]-2-imidazoline,
12) 5-chlor-4-(2-imidazoline-2-ylamino)-2,1,3-benzothiadiazol,
13) 2-(1,2,3,4-tétrahydro-5-naphtylamino)-2-imidazoline,
14) 2-(4-amino-2,6-dichlorophénylamino)-2-imidazoline,

15) 4-(2-imidazolin-2-ylamino)-2-méthyl-1,2-benzopyrazole,
16) 2-(3,4-dihydroxy-phénylamino)-2-imidazoline,
17) 2-amino-6-éthyl-4,5,7,8-tétrahydro-6H-oxazolo [5,4-d]-azépine,
18) 2-amino-6-(p-chlorobenzyl)-4,5,7,8-tétrahydro-6H-thiazolo[5,4-d]azépine,
19) 2-(4-brom-quinoxalin-5-ylamino)-2-imidazoline.

ainsi que leurs sels d'addition d'acide physiologiquement compatibles en tant qu'additifs de matières fourragères dans l'engraissement pour améliorer la croissance du bétail et la mise en valeur du fourrage.

5. Procédé pour la préparation d'un nouveau fourrage, caractérisé en ce qu'un fourrage ordinaire est mélangé à un composé selon les revendications 1 à 4 en une concentration de 0,02 jusqu'à 1 000 ppm.

6. Utilisation d'un composé selon les revendications 1-4 sur des systèmes parentéraux avec libération retardée de la substance active pour augmenter la prise de poids quotidienne et pour améliorer la mise en valeur du fourrage dans l'engraissement du bétail, des dosages de 1 jusqu'à 1 000 µg/kg de poids corporel par jour étant atteint.

7. Fourrage, contenant un composé selon les revendications 1 à 4 en une concentration de 0,02 jusqu'à 1 000 ppm.